# FASCICULE DE BREVET EUROPEEN

(11) **EP 1 467 666 B1**
(45) Date de publication et mention de la délivrance du brevet: **17.02.2010**
(21) Numéro de dépôt: 02803823.0
(22) Date de dépôt: 19.11.2002
(51) Int. Cl.: A61B 17/70

(54) **CONNECTEUR POUR SYSTEME D'ANCRAGE VERTEBRAL**
VERBINDUNGSSTÜCK FÜR EIN WIRBELVERANKERUNGSSYSTEM
CONNECTOR FOR VERTEBRAL ANCHORING SYSTEM

(30) Priorité: 27.11.2001 FR 0115298
(43) Date de publication de la demande: 20.10.2004
(73) Titulaire: Surgiview, 75002 Paris (FR)
(72) Inventeur: MAZEL, Christian, 92100 Boulogne Billancourt (FR); POMMIER, Arnaud, F-59283 Raimbeaucourt (FR); LEROY, Jean-Yves, F-62870 Campagne les Hesdin (FR); LEROY, Eric, F-62223 Saint-Nicolas les Arras (FR)
(74) Mandataire: Schmitt, John
(86) Numéro de dépôt international: PCT/FR2002/003951
(87) Numéro de publication internationale: WO 2003/045261

(56) Documents cités:
- DE-A- 19 726 754
- DE-U- 29 818 831
- FR-A- 2 720 261
- FR-A- 2 734 471
- FR-A- 2 765 093

## Description

La présente invention est relative à un connecteur pour système d'ancrage vertébral permettant l'utilisation soit d'une vis poly axiale d'ancrage osseux à tête sphérique, soit d'une vis d'ancrage osseux à tête filetée.

Un système d'ancrage selon le préambule de la revendication 1 est connu du document DE-U-298 18 831.

On connaît d'après le brevet US 3 997 138 des systèmes d'ancrage vertébral pour ostéosynthèse rachidienne comportant des vis de fixation qui sont constituées d'une partie filetée d'ancrage osseux, d'une tête d'entraînement à six pans qui se prolonge, à l'opposé de la première partie filetée, par une seconde partie filetée à pas mécanique.

La tête à six pans de la vis de fixation comporte deux canaux parallèles disposés de part et d'autre de la seconde partie filetée, afin de recevoir respectivement une tige de liaison.

Le système d'ancrage vertébral comporte un élément de retenue de forme hexagonale qui est percé d'un alésage central qui coopère avec la seconde partie filetée de la vis de fixation et de deux canaux parallèles disposés de part et d'autre de l'alésage central.

Les canaux de l'élément de retenue sont positionnés de manière à coopérer avec ceux de la tête de la vis de fixation.

Le système d'ancrage vertébral comporte un écrou de serrage à profil hexagonal qui vient coopérer avec la seconde partie filetée de la vis d'ancrage pour bloquer, d'une part l'élément de retenue sur la tête de la vis et d'autre part, les tiges de liaison en translation et en rotation à l'intérieur de canaux parallèles.

On constate que le système d'ancrage vertébral ne permet pas le réglage angulaire de l'élément de retenue et donc des tiges de liaison par rapport à la vis de fixation.

Egalement, ce type de système d'ancrage est adapté uniquement pour venir se fixer sur des vis de fixation à tête filetée et ne peut pas recevoir d'autres vis de fixation telles que celles à tête sphérique ou poly axiale.

Le système d'ancrage vertébral suivant la présente invention a pour objet de comporter des connecteurs permettant de recevoir soit des vis poly axiales soit des vis à tête filetée et de permettre un réglage angulaire autour de la tête de la vis correspondante.

Le système d'ancrage vertébral pour ostéosynthèse rachidienne suivant la présente invention comporte des connecteurs comme définis dans la revendication 1.

Le système d'ancrage vertébral pour ostéosynthèse rachidienne suivant la présente invention comporte un manchon cylindrique dont le filetage interne coopère avec le moyen de serrage lors de la fixation de chaque connecteur sur une vis d'ancrage poly axiale.

Le système d'ancrage vertébral pour ostéosynthèse rachidienne suivant la présente invention comporte un manchon cylindrique dont la surface sphérique se prolonge en direction de l'extérieur dudit manchon par une seconde surface à profil conique dont la base la plus large est dirigée en direction de l'extérieur du premier l'élément.

Le système d'ancrage vertébral pour ostéosynthèse rachidienne suivant la présente invention comporte un manchon cylindrique dont la surface sphérique comprend à sa base un épaulement séparant cette dernière de la, surface conique, afin de constituer une portée ou une face d'appui à la vis de fixation correspondante.

Le système d'ancrage vertébral pour ostéosynthèse rachidienne suivant la présente invention comporte un manchon cylindrique dont la partie haute, constituant l'extrémité libre, présente une face plane formant une face d'appui aux moyens de serrage lors de la fixation de chaque connecteur sur une vis d'ancrage à tête filetée.

Le système d'ancrage vertébral pour ostéosynthèse rachidienne suivant la présente invention comporte des moyens de serrage qui sont constitués d'un bouchon fileté à empreinte interne permettant la fixation de chaque connecteur sur des vis d'ancrage poly axiales à tête sphérique.

Le système d'ancrage vertébral pour ostéosynthèse rachidienne suivant la présente invention comporte des moyens de serrage qui sont constitués d'un écrou de serrage à empreinte externe permettant la fixation de chaque connecteur sur des vis d'ancrage à tête filetée.

Le système d'ancrage vertébral pour ostéosynthèse rachidienne suivant la présente invention comporte un manchon cylindrique pourvu, sur sa face externe et sur toute sa hauteur, de deux méplats parallèles.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une vue en perspective éclatée illustrant un connecteur pour système d'ancrage vertébral à vis poly axiale suivant la présente invention.
Figure 2 est une vue en perspective montrant le connecteur pour système d'ancrage vertébral à vis poly axiale en position montée.
Figures 3 et 4 sont des vues en perspective montrant en détail l'un des éléments du connecteur pour système d'ancrage vertébral suivant la présente invention.
Figure 5 est une vue en perspective éclatée représentant un connecteur pour système d'ancrage vertébral à vis à double ancrage suivant la présente invention.

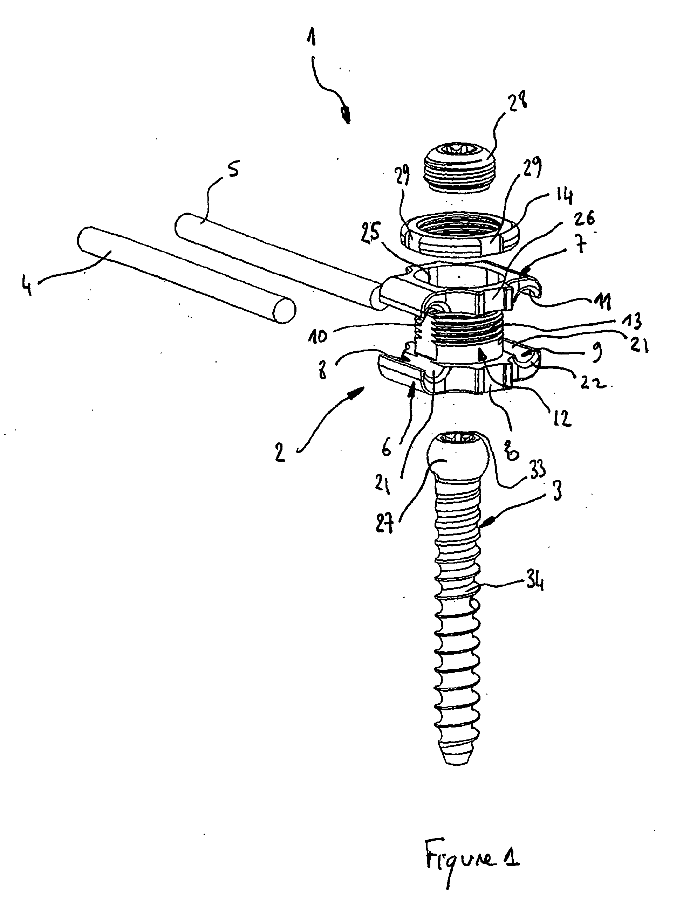

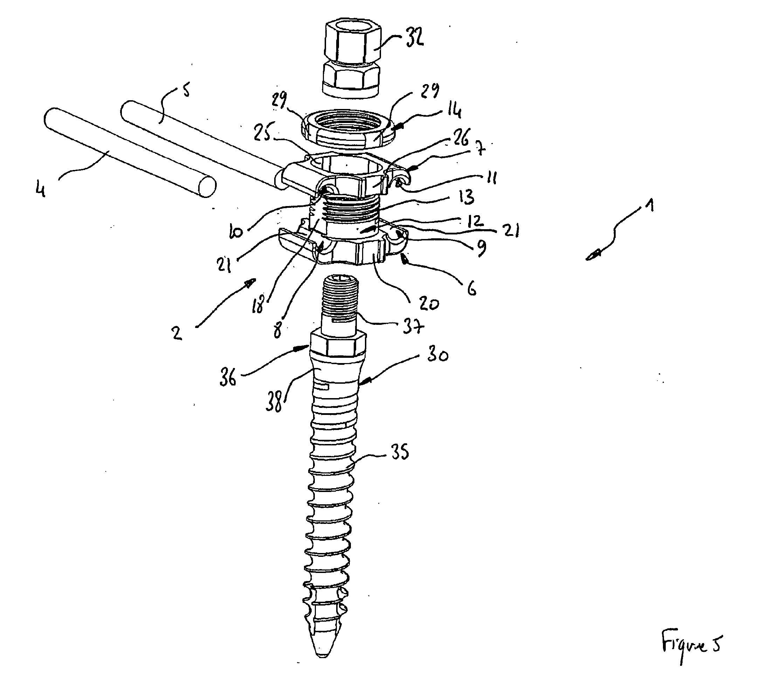

On a montré en figures 1 et 5, un système d'ancrage vertébral 1 comportant un connecteur 2 destiné à coopérer avec une vis poly axiale 3 ou une vis à tête filetée 30 pour l'immobilisation de deux tiges de liaison 4 et 5.

Le connecteur 2 est constitué d'un premier élément inférieur 6 coopérant avec un second élément supérieur 7 pourvu chacun de logement 8, 9, 10, et 11 en portion de cylindre pour la réception et l'immobilisation des tiges de liaison 4 et 5 au moyen d'un écrou de serrage 14.

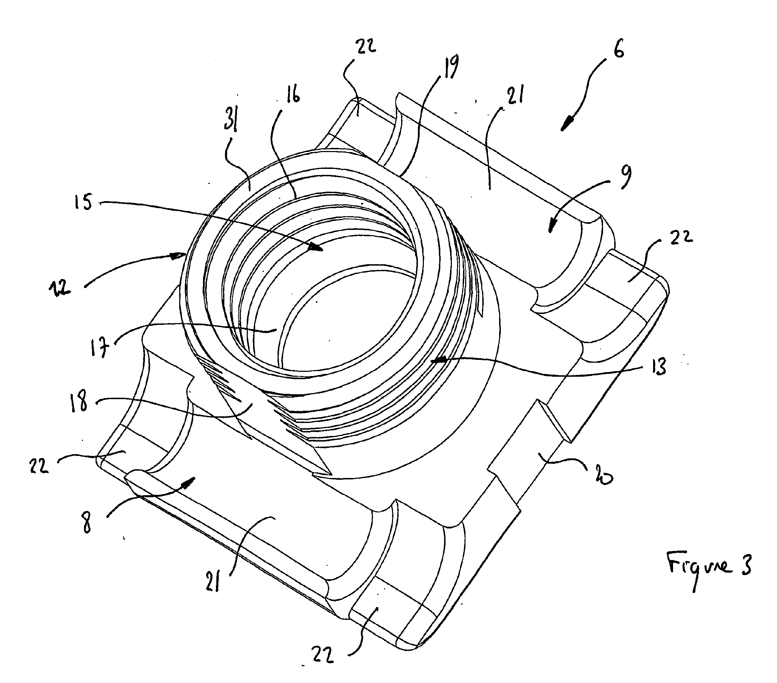

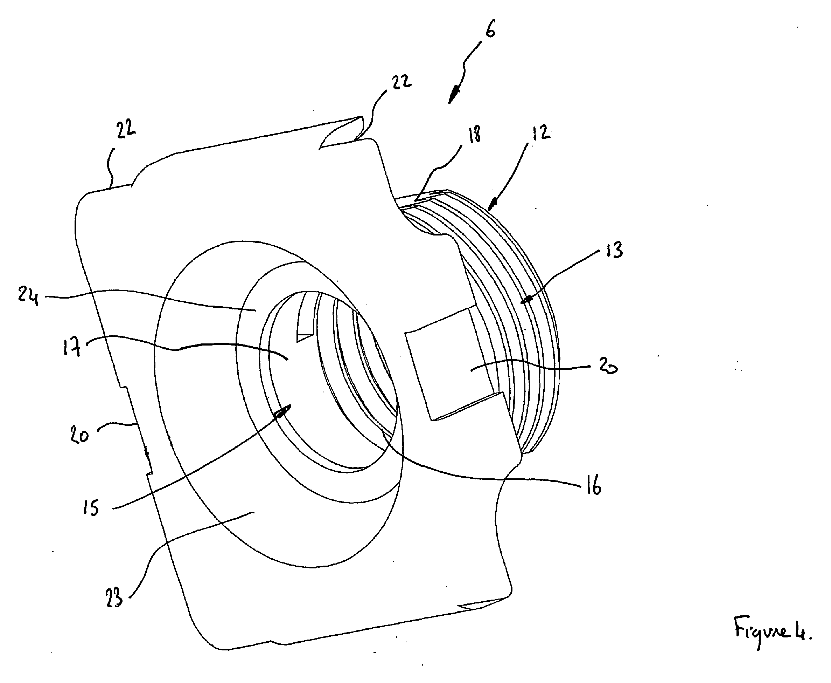

On a représenté en figures 3 et 4, le premier élément 6 du connecteur 2 qui est constitué d'une cheminée ou manchon cylindrique creux 12 qui s'étend suivant une direction verticale et perpendiculaire aux logements 8 et 9.

Les logements en portion de cylindre 8 et 9 de l'élément 6 sont ménagés de part et d'autre du manchon cylindrique 12 et parallèlement l'un à l'autre.

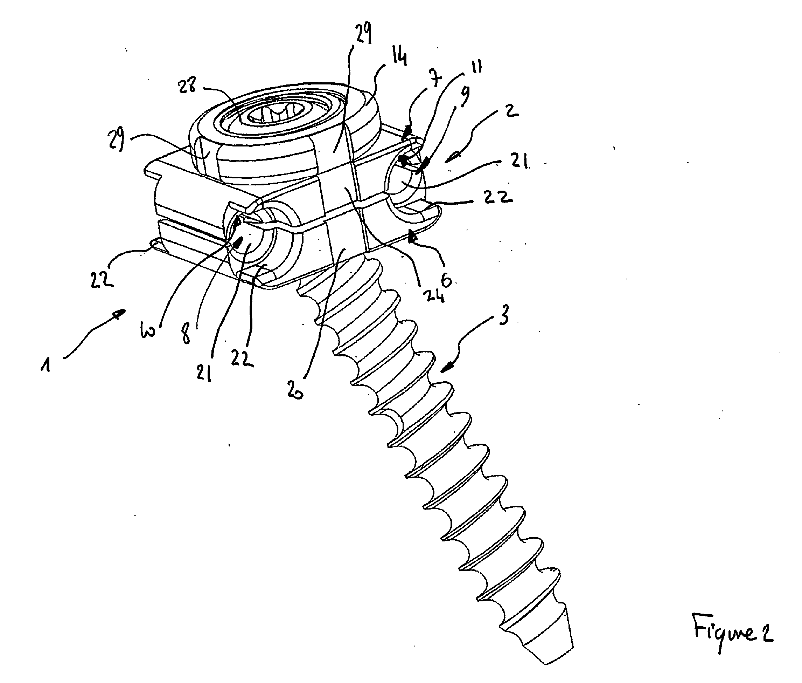

Le manchon cylindrique creux 12 présente sur sa face externe un filetage 13 qui coopère, lors du montage du système d'ancrage 1, avec l'écrou de serrage 14 pour l'immobilisation d'une part, du second élément 7 sur le premier 6 et d'autre part, des tiges de liaison 4 et 5 dans les logements 8, 9, 10 et 11 (figure 2).

L'écrou de serrage 14 comporte, sur son pourtour externe, des encoches 29 permettant son entraînement en rotation autour du filetage externe 13 du manchon 12.

Le manchon cylindrique 12 présente dans sa partie interne un alésage débouchant 15 pourvu d'un filetage 16, d'une première surface 17 en portion de sphère et d'une seconde surface 23 à profil conique.

Le filetage interne 16 coopère, lors de l'utilisation d'une vis poly axiale 3, avec un bouchon fileté 28 à empreintes internes permettant l'immobilisation et le blocage dans une position déterminée de ladite vis 3 (figure 2).

La surface sphérique 17 se prolonge en direction de l'extérieur du manchon cylindrique 12 par la seconde surface 23 à profil conique dont la base la plus large est dirigée en direction de l'extérieur de l'élément 6.

Les surfaces sphériques 17 et coniques 23 sont positionnées à la base de la cheminée ou du manchon cylindrique 12, c'est à dire au niveau des logements en portion de cercle 8 et 9 ménagés dans l'élément 6.

La surface sphérique 17 comporte à sa base, c'est à dire à l'opposé du filetage interne 16, un épaulement 24 séparant cette dernière de la surface conique 23, afin de constituer une portée ou une face d'appui à la vis de fixation 3 et 30.

Le manchon cylindrique 12 comporte, sur sa face externe et sur toute sa hauteur, deux méplats parallèles 18 et 19, de manière à tronquer le filetage 13.

Les deux méplats 18 et 19 permettent de réduire le diamètre externe du manchon cylindrique 12 pour éviter à ce dernier de déborder dans les logements en portion de cylindre 8 et 9 de l'élément 6.

La partie haute, formant l'extrémité libre de la cheminée ou du manchon cylindrique 12, présente une face plane 31 servant d'appui à un écrou de serrage 32 à empreintes externes lors de l'utilisation d'une vis à tête filetée 30 (figure 5).

Le premier élément 6 comporte entre les logements en portion de cylindre 8 et 9 des empreintes 20 positionnées suivant une direction verticale et parallèle à l'axe principal de l'alésage interne 15 du manchon 12.

Les logements 8 et 9 comportent une portée 21 en portion de cylindre qui est délimitée, à chaque extrémité, par des épaulements 22 situés en dessous du profil en portion de cercle de la portée 21.

Ainsi, les portées en portion de cylindre 21 de chaque logement 8 et 9 présentent une longueur qui est inférieure à celle de l'élément 6 pour garantir un positionnement et une immobilisation parfaite des tiges de liaison 4 et 5 sur une distance suffisante.

Le second élément 7 comporte, entre les logements 10 et 11 en portion de cylindre, un alésage 25 qui est traversé par la cheminée ou le manchon cylindrique 12 du premier élément 6 (figure 1).

Le second élément 7 comporte, entre les logements en portion de cylindre 10 et 11, des empreintes 26 positionnées suivant une direction verticale et parallèle à l'axe principal de l'alésage 25 (figure 1).

Les logements 10 et 11 sont semblables à ceux 8 et 9 du premier élément 6 de manière à présenter une portée en portion de cylindre qui est délimitée, à chaque extrémité, par des épaulements situés en dessous du profil en portion de cylindre de la portée.

Les logements 8, 9, 10 et 11 de chaque élément 6, 7 sont destinés à recevoir respectivement les tiges de liaison 4 et 5 pour pouvoir les positionner et les immobiliser en rotation et en translation à l'intérieur de chaque élément 6, 7 du connecteur 2, lors du serrage de l'écrou 14 sur le manchon 12.

En figure 2, on a illustré le connecteur 2 suivant la présente invention en position montée et immobilisé sur une vis poly axiale 3.

La vis poly axiale 3 comporte une tête sphérique 27 pourvue d'une empreinte interne 33, pour l'entraînement en rotation et l'ancrage de ladite vis dans le corps vertébral correspondant.

La tête sphérique 27 de la vis poly axiale 3 se prolonge par un corps fileté 34 dont le filetage présente un filet progressif et tranchant en direction de la pointe de ladite vis.

La vis poly axiale 3 est introduite, avant son ancrage dans le corps vertébral, dans l'alésage interne 15 du manchon cylindrique 12 du premier élément 6, afin que sa tête sphérique 27 vienne en appui sur la face de la portée 24 qui est tournée du côté de la surface sphérique 17.

Après l'ancrage de la vis 3 dans le corps vertébral correspondant, le second élément 7 est immobilisé sur le premier 6 à l'aide de l'écrou 14 qui coopère avec le filetage externe 13 du manchon 12. L'immobilisation du second élément 7 entraîne simultanément le blocage des tiges de liaison 4 et 5 dans les logements 8, 9,10 et 11 correspondants du connecteur 2.

La surface conique 24 prévue dans l'alésage interne 15 du manchon cylindrique 12 permet un réglage angulaire du connecteur 2 autour de la tête sphérique 27 de la vis poly axiale 3.

Le connecteur 2 est immobilisé dans sa position angulaire autour de la tête 27 de la vis poly axiale par l'intermédiaire du bouchon 28, qui est vissé à l'intérieur du manchon cylindrique 12.

En figure 5, on a représenté le connecteur 2 suivant la présente invention monté sur une vis à tête filetée 30.

La vis 30 comporte une première partie filetée d'ancrage osseux 35, une tête d'entraînement 36 et dans le prolongement de la tête une seconde partie filetée 37 qui coopère avec un écrou de serrage 32.

La tête d'entraînement 36 comporte une portion lisse à profil évasé 38 située entre la partie filetée d'ancrage osseux 35 et ladite tête. Cette dernière présente un profil externe hexagonal pour l'entraînement en rotation de la vis 30.

La vis à tête filetée 30 est ancrée dans le corps vertébral correspondant avant de recevoir le premier élément 6 du connecteur 2. Le premier élément 6 est positionné sur la vis 30 de manière que la tête 36 vienne en appui sur la face de l'épaulement 24 tournée du coté de la surface conique 23 ménagée dans le manchon cylindrique 12.

Ensuite, le second élément 7 est immobilisé sur le premier 6 à l'aide de l'écrou 14 qui coopère avec le filetage externe 13 du manchon 12. L'immobilisation du second élément 7 entraîne simultanément le blocage des tiges de liaison 4 et 5 dans les logements 8, 9,10 et 11 correspondants du connecteur 2.

Le connecteur 2 est immobilisé sur la vis 30 par l'intermédiaire de l'écrou de serrage 32 qui est vissé sur la seconde partie filetée 37 jusqu'à son blocage contre la face plane 31 du manchon cylindrique 12.

II doit d'ailleurs être entendu que la description qui précède n'a été donnée qu'à titre d'exemple et qu'elle ne limite nullement le domaine de l'invention.

## Revendications

1. Système d'ancrage vertébral pour ostéosynthèse rachidienne comportant des connecteurs (2) permettant, sur chaque vis d'ancrage vertébral (3, 30), la fixation de tiges de liaison parallèles (4, 5), chaque connecteur (2) comprenant un premier élément inférieur (6) pourvu de logements parallèles en portion de cylindre (8, 9) recevant respectivement la tige de liaison (4, 5) correspondante, ledit premier élément inférieur (6) comportant un manchon cylindrique creux fileté (12) s'étendant verticalement entre lesdits logements (8, 9), **caractérisé en ce que** chaque connecteur (2) comporte :
❖ un second élément supérieur (7) comprenant des logements parallèles en portion de cylindre (10, 11) recevant respectivement la tige de liaison (4, 5) correspondante et un alésage (25) disposé entre lesdits logements (10, 11) pour être traversé par le manchon cylindrique (12) du premier élément inférieur (6),
❖ un écrou de serrage (14) coopérant avec un filetage externe (13) du manchon cylindrique (12) du premier élément inférieur (6) pour la fixation d'une part, du second élément supérieur (7) sur le premier (6) et d'autre part, des tiges de liaison (4, 5) dans les logements (8, 9; 10, 11),
❖ et des moyens de serrage (28, 32) coopérant avec la partie interne du manchon cylindrique (12) du premier élément inférieur (6), ladite partie interne étant constituée d'un alésage débouchant (15) pourvu d'un filetage (16), d'une première surface (17) en portion de sphère et d'une seconde surface (23) à profil conique afin d'assurer la fixation de chaque connecteur (2) sur la vis d'ancrage (3, 30) correspondante.

2. Système d'ancrage vertébral pour ostéosynthèse rachidienne suivant la revendication 1, **caractérisé en ce que** le filetage interne (16) du manchon cylindrique (12) coopère avec le moyen de serrage (28) lors de la fixation de chaque connecteur (2) sur une vis d'ancrage poly axiale (3).

3. Système d'ancrage vertébral pour ostéosynthèse rachidienne suivant la revendication 1, **caractérisé en ce que** la surface sphérique (17) se prolonge en direction de l'extérieur du manchon cylindrique (12) par la seconde surface (23) à profil conique dont la base la plus large est dirigée en direction de l'extérieur de l'élément (6).

4. Système d'ancrage vertébral pour ostéosynthèse rachidienne suivant la revendication 1, **caractérisé en ce que** la surface sphérique (17) comporte à sa base, un épaulement (24) séparant cette dernière de la surface conique (23), afin de constituer une portée ou une face d'appui à la vis de fixation (3, 30) correspondante.

5. Système d'ancrage vertébral pour ostéosynthèse rachidienne suivant la revendication 1, **caractérisé en ce que** la partie haute, formant l'extrémité libre du manchon cylindrique (12), présente une face plane (31) formant une face d'appui aux moyens de serrage (32) lors de la fixation de chaque connecteur (2) sur une vis d'ancrage à tête filetée (30).

6. Système d'ancrage vertébral pour ostéosynthèse rachidienne suivant la revendication 1, **caractérisé en ce que** le manchon cylindrique (12) du premier élément (6) comporte sur sa face externe et sur toute sa hauteur deux méplats parallèles (18, 19).

## Claims

1. Vertebral anchoring system for spinal osteosynthesis, with connectors (2) allowing parallel connection rods (4, 5) to be fixed on each vertebral anchoring screw (3, 30), each connector (2) comprising a first, lower element (6) provided with parallel seats (8, 9) in the shape of a segment of a cylinder, each receiving the corresponding connection rod (4, 5), said first, lower element (6) comprising a threaded hollow cylindrical sleeve (12) extending vertically between said seats (8, 9), **characterized in that** each connector (2) comprises:
- a second, upper element (7) comprising parallel seats (10, 11) in the shape of a segment of a cylinder, each receiving the corresponding connection rod (4, 5), and a bore (25) arranged between said seats (10, 11) so as to be traversed by the cylindrical sleeve (12) of the first, lower element (6),
- a clamping nut (14) cooperating with an external thread (13) of the cylindrical sleeve (12) of the first, lower element (6) in order, on the one hand, to fix the second, upper element (7) on the first (6), and, on the other hand, to fix the connection rods (4, 5) in the seats (8, 9, 10, 11),
- and clamping means (28, 32) cooperating with the inner part of the cylindrical sleeve (12) of the first, lower element (6), said inner part consisting of a through-bore (15) provided with a thread (16), a first surface (17) in the shape of a segment of a sphere, and a second surface (23) of conical profile so as to fix each connector (2) on the corresponding anchoring screw (3, 30).

2. Vertebral anchoring system for spinal osteosynthesis according to Claim 1, **characterized in that** the internal thread (16) of the cylindrical sleeve (12) cooperates with the clamping means (28) upon fixation of each connector (2) on a multi-axial anchoring screw (3).

3. Vertebral anchoring system for spinal osteosynthesis according to Claim 1, **characterized in that** the spherical surface (17) is continued, in the direction of the outside of the cylindrical sleeve (12), by the second surface (23) of conical profile whose widest base is oriented in the direction of the outside of the element (6).

4. Vertebral anchoring system for spinal osteosynthesis according to Claim 1, **characterized in that** the spherical surface (17) has, at its base, a shoulder (24) separating the latter from the conical surface (23), so as to constitute a support or bearing face for the corresponding fixation screw (3, 30).

5. Vertebral anchoring system for spinal osteosynthesis according to Claim 1, **characterized in that** the upper part, forming the free end of the cylindrical sleeve (12), has a plane face (31) forming a bearing face for the clamping means (32) upon fixation of each connector (2) on an anchoring screw with threaded head (30).

6. Vertebral anchoring system for spinal osteosynthesis according to Claim 1, **characterized in that** the cylindrical sleeve (12) of the first element (6) has, on its outer face and over its entire height, two parallel flat surfaces (18, 19).

## Patentansprüche

1. Wirbelverankerungssystem für die Wirbelsäulenosteosynthese mit Verbindungsstücken (2), die an jeder Wirbelverankerungsschraube (3, 30) die Fixierung von parallelen Verbindungsstangen (4, 5) gestatten, wobei jedes Verbindungsstück (2) ein erstes unteres Element (6) umfasst, das mit parallelen Aufnahmen (8, 9) mit Zylindersegmentform versehen ist, die jeweils die entsprechende Verbindungsstange (4, 5) aufnehmen, wobei das erste untere Element (6) eine zylindrische, hohle Gewindebuchse (12) umfasst, die sich vertikal zwischen den Aufnahmen (8, 9) erstreckt, **dadurch gekennzeichnet, dass** jedes Verbindungsstück (2) Folgendes umfasst:
• ein zweites oberes Element (7), das parallele Aufnahmen (10, 11) mit Zylindersegmentform, die jeweils die entsprechende Verbindungsstange (4, 5) aufnehmen, und eine zwischen den Aufnahmen (10, 11) angeordnete Bohrung (25) umfasst, die von der zylindrischen Buchse (12) des ersten unteren Elements (6) durchquert wird,
• eine Festspannmutter (14), die zur Fixierung einerseits des zweiten oberen Elements (7) am ersten (6) und andererseits der Verbindungsstangen (4, 5) in den Aufnahmen (8, 9; 10, 11) mit einem äußeren Gewinde (13) der zylindrischen Buchse (12) des ersten unteren Elements (6) zusammenwirkt,
• und Festspannmittel (28, 32), die mit dem Innenteil der zylindrischen Buchse (12) des ersten unteren Elements (6) zusammenwirken, wobei der Innenteil aus einer mit einem Gewinde (16) versehenen Durchgangsbohrung (15), einer ersten Oberfläche (17) mit Kugelsegmentform und einer zweiten Oberfläche (23) mit konischem Profil besteht, um die Fixierung jedes Verbindungsstücks (2) an der entsprechenden Verankerungsschraube (3, 30) zu gewährleisten.

2. Wirbelverankerungssystem für die Wirbelsäulenosteosynthese nach Anspruch 1, **dadurch gekennzeichnet, dass** das innere Gewinde (16) der zylindrischen Buchse (12) bei der Fixierung jedes Verbindungsstücks (2) an einer multiaxialen Verankerungsschraube (3) mit dem Festspannmittel (28) zusammenwirkt.

3. Wirbelverankerungssystem für die Wirbelsäulenosteosynthese nach Anspruch 1, **dadurch gekennzeichnet, dass** sich die kugelförmige Oberfläche (17) mittels der zweiten Oberfläche (23) mit konischem Profil, deren breiteste Basis in Richtung der Außenseite des Elements (6) weist, in Richtung der Außenseite der zylindrischen Buchse (12) fortsetzt.

4. Wirbelverankerungssystem für die Wirbelsäulenosteosynthese nach Anspruch 1, **dadurch gekennzeichnet, dass** die kugelförmige Oberfläche (17) an ihrer Basis einen Absatz (24) umfasst, der sie von der konischen Oberfläche (23) trennt und so eine Auflage- oder eine Stützfläche für die entsprechende Fixierungsschraube (3, 30) darstellt.

5. Wirbelverankerungssystem für die Wirbelsäulenosteosynthese nach Anspruch 1, **dadurch gekennzeichnet, dass** der obere Teil, der das freie Ende der zylindrischen Buchse (12) bildet, eine ebene Fläche (31) aufweist, die bei der Fixierung jedes Verbindungsstücks (2) an einer Verankerungsschraube (30) mit Gewindekopf eine Stützfläche für die Festspannmittel (32) bildet.

6. Wirbelverankerungssystem für die Wirbelsäulenosteosynthese nach Anspruch 1, **dadurch gekennzeichnet, dass** die zylindrische Buchse (12) des ersten Elements (6) auf ihrer Außenfläche und über ihre ganze Höhe zwei parallele Abflachungen (18, 19) umfasst.
